# EUROPEAN PATENT SPECIFICATION

(11) **EP 3 640 015 B1**
(45) Date of publication and mention of the grant of the patent: **17.02.2021**
(21) Application number: 18200862.3
(22) Date of filing: 17.10.2018
(51) Int. Cl.: B29D 23/00, B29L 31/00, B29L 23/00, B29K 105/00, A61M 1/36, B01D 61/00, B01D 63/02, B23B 5/08

(54) **PROCESS FOR MANUFACTURING A MEDICAL DEVICE HOUSING**
VERFAHREN ZUR HERSTELLUNG EINES GEHÄUSES EINER MEDIZINISCHEN VORRICHTUNG
PROCÉDÉ DE FABRICATION D'UN BOÎTIER D'UN DISPOSITIF MÉDICAL

(43) Date of publication of application: 22.04.2020
(73) Proprietor: Gambro Lundia AB, 220 10 Lund (SE)
(72) Inventor: Wagner, Steffen, 72469 Meßstetten (DE); Klink, Bjoern, 72414 Rangendingen (DE); Wiest, Jochen, 72401 Haigerloch-Gruol (DE); Esse, Sergej, 72406 Bisingen (DE)
(74) Representative: Perchenek, Nils

(56) References cited:
- JP-A- 2001 212 701
- US-A1- 2011 166 527

## Description

### Technical Field

The present disclosure relates to a process for manufacturing a rotationally symmetric plastic housing for a medical device, e.g., a dialyzer, an ultrafilter, or a device for removing carbon dioxide from blood.

### Description of the Related Art

Common processes for manufacturing cylindrical housings for medical products, such as dialyzers, involve an injection molding process. Because an injection molding tool must be manufactured for each housing design, the housing is very expensive when only a small number of housings are produced. Geometrical changes cannot be implemented in the housing design within a short timeframe, because the injection molding tool must be altered. Different polymer materials cannot be tested by using the same injection molding tool, because these tools are designed for one specific polymer material. Parts designed for production by injection molding must have uniform wall thickness over the whole part to avoid shrink holes.

Small numbers of housings may also be manufactured by a 3D printing process. However, medical grade polymers are not generally available as starting materials for 3D printing. Moreover, 3D printed parts usually exhibit high surface roughness, which is undesirable for medical devices.

US 2011/166527 A1 discloses the manufacture of parts of a medical device on a lathe and milling machine. JP 2001 212701 A discloses lathe turning of a hollow cylindrical part using a mandrel.

It would be desirable to provide a manufacturing process for medical device housings, which uses medical grade polymers, yields housings with a high surface quality as required for medical devices, and allows for lowering production cost even if only a small number of housings is produced.

### Summary

The present disclosure provides a process for manufacturing a rotationally symmetric plastic housing for a medical device. The process involves the use of a CNC lathe and produces a housing having low surface roughness.

### Brief Description of the Drawings

- Figure 1: is a schematic cross sectional view of a semifinished housing and some of the components involved in an embodiment of the process of the present disclosure;
- Figure 2: is a schematic cross sectional view of an assembly of the components shown in Figure 1.

### Detailed Description

The present disclosure provides a process for manufacturing a rotationally symmetric plastic housing for a medical device. Examples of medical devices include capillary dialyzers, hemofilters, plasma filters, ultrafilters, and gas exchangers. In one embodiment, the medical device is a device for extracorporeal membrane oxygenation (ECMO) or for extracorporeal carbon dioxide removal (ECCO₂R). The process for manufacturing a rotationally symmetric plastic housing for a medical device of the present disclosure comprises the steps of:
a) providing a massive cylindrical rod of a medical grade polymer;
b) producing a rotationally symmetric bore along the longitudinal axis of the cylindrical rod, the bore extending from a first end of the cylindrical rod to a second, opposite end of the cylindrical rod, wherein the diameter of the bore decreases from the first end in the direction of the second end;
c) introducing a mandrel having a shape matching the inner contour of the bore into the bore from the first end of the rod;
d) mounting a fixation cover on the second end of the rod;
e) connecting the mandrel and the fixation cover by a fastener to press the rod onto the mandrel;
f) machining the outer surface of the rod using a CNC lathe to produce a desired outer contour of the housing.

The process involves providing a massive cylindrical rod of a medical grade polymer. Examples of suitable medical grade polymers include polycarbonate (PC), polyethylene terephthalate (PET), polymethylmethacrylate (PMMA), or polypropylene (PP). In one embodiment, the medical grade polymer is polycarbonate.

In a first step of the manufacturing process, a rotationally symmetric bore is produced in the polymer rod along the longitudinal axis of the rod. The bore can be produced using a suitable cutting tool, e.g., a milling cutter or a CNC milling machine. At this stage of the manufacturing process, the polymer rod can withstand the forces induced by the cutting tools producing the bore.

The diameter of the bore of the housing tapers off from a first end of the polymer rod in the direction of the other end of the polymer rod. In one embodiment of the process, the gradient of the diameter of the bore is constant over the length of the bore, i.e., the bore takes the form of a truncated cone. In a particular embodiment, the angle of aperture of the cone is in the range of from 2 to 4 degrees. In another embodiment, the gradient of the diameter of the bore varies over the length of the bore, and zones having higher gradient alternate with zones having lower gradient. As a result, the bore takes a generally frusto-conical form with one or more ledges.

In a particular embodiment, the diameter of the bore of the housing tapers off from a first end of the polymer rod in the direction of the other end of the polymer rod; reaches a minimum at a distance from the other end; and then increases again, either at a constant gradient, or at a varying gradient, so that the diameter of the bore at the other end is larger than the minimum diameter.

In a second step, a mandrel having a shape matching the shape of the bore is introduced into the bore from the first end of the rod, i.e., where the diameter of the bore is largest, thereby mounting and fastening the polymer rod on the mandrel. The inside surface of the bore contacts the surface of the mandrel.

A fixation cover is mounted on the other end of the rod. The mandrel and the fixation cover are connected to each other and tied together by suitable fasteners. In one embodiment, one or more bores are provided in the fixation cover, and corresponding threaded bores are provided in the end face of the mandrel. A screw is introduced into each threaded bore of the mandrel through the corresponding bore of the fixation cover, and the fixation cover and the mandrel are screwed together. Thus, the polymer rod is pressed onto the mandrel.

In one embodiment, the fixation cover is disc-shaped and has a central bore, i.e., it takes the form of a plain washer. In another embodiment, the fixation cover is disc-shaped and has several bores equidistant to the center of the disc. In one embodiment, the diameter of the fixation cover is constant over its entire height. The diameter is larger than the diameter of the bore at the end of the polymer rod the fixation cover is mounted to. In another embodiment, the diameter of the fixation cover varies over its height, to match the shape of the bore at the end of the polymer rod the fixation cover is mounted to. This embodiment can be used in cases where, viewed along the longitudinal axis of the polymer rod, the diameter of the bore reaches a minimum and then increases again towards the end of the polymer rod the fixation cover is mounted to. The fixation cover is introduced into the bore, and the inside surface of the bore contacts the surface of the fixation cover, further stabilizing the polymer rod from inside. This embodiment allows for more flexibility in designing the inner contour of the housing.

In one embodiment, the mandrel and the fixation cover are comprised of metal. Examples of suitable metals include aluminum, stainless steel, and brass.

After the polymer rod has been mounted on the mandrel, the mandrel is transferred to a CNC lathe. The outer surface of the polymer rod then is machined using the CNC lathe to produce the desired outer contour of the housing. The outside surface of the polymer rod can be machined without a risk of breaking the material, as the rod is stabilized on both ends and from within by the mandrel and the fixing cover. Furthermore, vibrations induced by the cutting tool are minimized by this stabilization, resulting in a smoother surface of the final housing.

In one embodiment of the process, the arithmetic average roughness Rₐ, measured according to DIN EN ISO 4287:2010, of the wall surface of the finished housing is less than 10 µm, for instance, in the range of from 0.2 µm to 5.0 µm, or even from 0.2 µm to 2.0 µm. Both the inner and the outer surface of the finished housing show an arithmetic average roughness Rₐ in the aforementioned range. In contrast to that, parts produced by 3D printing generally exhibit much higher surface roughness, typically with an Rₐ in the range of from 12.5 µm to 25 µm.

The process of the present disclosure allows to produce housings having a small wall thickness, i.e. thin-walled housings. A wall thickness of less than 1 mm can be achieved. In one embodiment, the wall thickness of the final housing is in the range of from 0.5 mm to 5 mm, for instance, from 0.5 mm to 2.0 mm.

In one embodiment, the wall thickness is uniform over the whole length of the housing. In another embodiment, at least one section of the final housing has a wall thickness that is different from the wall thickness of other sections of the housing. In other words, the wall thickness of the housing can vary over its length. The process of the present disclosure thus is not limited to producing housings with uniform wall strength as are injection molding processes. This allows for more flexibility in designing the housing.

Another advantage of the process of the present disclosure is that it can use polymer materials that normally cannot be processed by injection molding. Examples include non-thermoplastic polymers like polytetrafluoroethylene (Teflon®), silicone rubber, and polyurethane (PUR).

It will be understood that the features mentioned above and those described hereinafter can be used not only in the combination specified but also in other combinations or on their own, without departing from the scope of the present invention.

The process of the present disclosure will now be further described in the following examples and referring to the attached drawings.

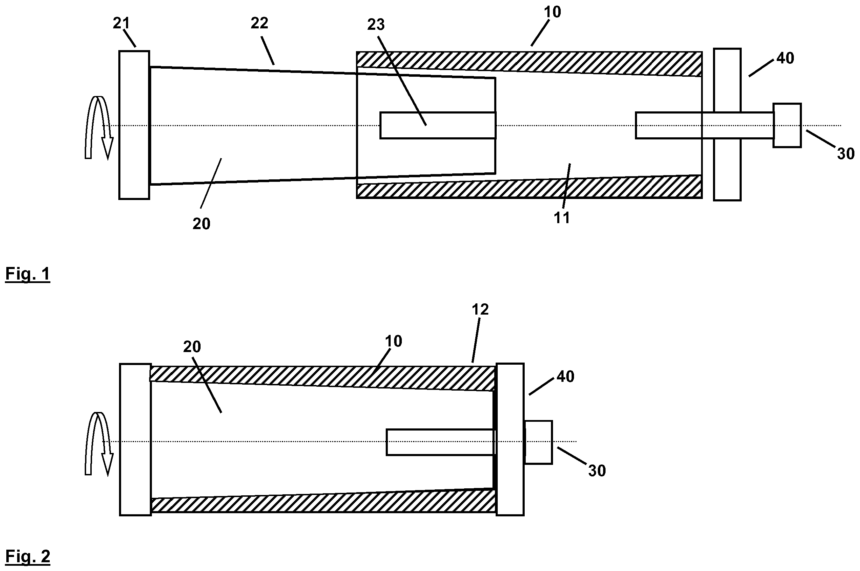

Figure 1 is a schematic cross sectional view of a semi-finished housing 10 and some of the components involved in an embodiment of the process of the present disclosure. A mandrel 20 having a disc-shaped base 21, a frusto-conical part 22 matching the inner contour of a bore 11 in the semi-finished housing 10, and a threaded bore 23 in its end face is shown. The mandrel 20 is introduced into the bore 11 from the end of the housing 10 where the bore 11 has its largest diameter. A fixation cover 40 is mounted on the opposite end of the housing 10. In the embodiment shown, the fixation cover 40 is disc-shaped and has a central bore matching the threaded bore 23 of the mandrel 20. A screw 30 is used to connect the mandrel 20 and the fixation cover 40 and tie them together.

Figure 2 is a schematic cross sectional view of an assembly of the components shown in Figure 1. The housing 10 is pinned on the mandrel 20 and fastened by the screw 30 and the fixation cover 40. The assembly as shown in Fig. 2 is transferred to a CNC lathe and the outer surface 12 of the housing 10 is machined to yield a finished housing having a desired rotationally symmetric outer contour.

### List of reference signs

- 10: housing
- 11: bore
- 12: outer surface
- 20: mandrel
- 21: base
- 22: truncated cone
- 23: threaded bore
- 30: screw
- 40: fixation cover

## Claims

1. A process for manufacturing a rotationally symmetric plastic housing (10) for a medical device, comprising
a) providing a massive cylindrical rod of a medical grade polymer;
b) producing a rotationally symmetric bore (11) along the longitudinal axis of the cylindrical rod, the bore (11) extending from a first end of the cylindrical rod to a second, opposite end of the cylindrical rod, wherein the diameter of the bore (11) decreases from the first end in the direction of the second end;
c) introducing a mandrel (20) having a shape matching the inner contour of the bore (11) into the bore (11) from the first end of the rod;
d) mounting a fixation cover (40) on the second end of the rod;
e) connecting the mandrel (20) and the fixation cover (40) by a fastener (30) to press the rod onto the mandrel (20) ;
f) machining the outer surface (12) of the rod using a CNC lathe to produce a desired outer contour of the housing (10) .

2. The process of claim 1, wherein the medical grade polymer is selected from the group consisting of polycarbonate, polyethylene terephthalate, polymethylmethacrylate, and polypropylene.

3. The process of claim 2, wherein the medical grade polymer is polycarbonate.

4. The process of claim 1, wherein the medical grade polymer is a non-thermoplastic polymer.

5. The process of claim 4, wherein the medical grade polymer is selected from the group consisting of polytetrafluoroethylene, silicone rubber, and polyurethane.

6. The process of any one of claims 1 to 5, wherein the mandrel (20) comprises a disc-shaped base (21), a truncated cone (22) connected to the base (21), and at least one threaded bore (23) in a face of the truncated cone (22) opposite to the base (21).

7. The process of any one of claims 1 to 6, wherein the fixation cover (40) is disc-shaped and has several bores equidistant to the center of the disc.

8. The process of any one of claims 1 to 7, wherein the fastener (30) comprises at least one screw.

9. The process of any one of claims 1 to 8, wherein the bore (11) has the form of a truncated cone with an angle of aperture in the range of from 2° to 4°.

10. The process of any one of claims 1 to 9, wherein the wall thickness of the housing (10) after step f) is in the range of from 0.5 to 5 mm.

11. The process of any one of claims 1 to 10, wherein the wall thickness of the housing (10) after step f) varies over the length of the housing.

12. The process of any one of claims 1 to 11, wherein the arithmetic average roughness Rₐ of the wall surface of the housing (10), measured according to DIN EN ISO 4287, after step f) is in the range of 0.2 µm to 5.0 µm.

13. The process of any one of claims 1 to 12, wherein the medical device is a capillary dialyzer, a hemofilter, a plasma filter, or an ultrafilter.

14. The process of any one of claims 1 to 12, wherein the medical device is a device for extracorporeal membrane oxygenation or a device for extracorporeal carbon dioxide removal.

## Patentansprüche

1. Verfahren zur Herstellung eines rotationssymmetrischen Kunststoffgehäuses (10) für ein Medizinprodukt, umfassend
a) Bereitstellen eines massiven zylindrischen Stabs eines für medizinische Anwendung geeigneten Polymers;
b) Erzeugen einer rotationssymmetrischen Bohrung (11) entlang der Längsachse des zylindrischen Stabs, wobei die Bohrung (11) sich von einem ersten Ende des zylindrischen Stabs bis zu einem zweiten, gegenüberliegenden Ende des zylindrischen Stabs erstreckt, wobei der Durchmesser der Bohrung (11) vom ersten Ende in Richtung des zweiten Endes abnimmt;
c) Einbringen eines Dorns (20) mit einer Form, die der inneren Kontur der Bohrung (11) entspricht, in die Bohrung (11) vom ersten Ende der Stange her;
d) Montieren einer Befestigungsabdeckung (40) am zweiten Ende der Stange;
e) Verbinden des Dorns (20) und der Befestigungsabdeckung (40) durch ein Verbindungselement (30), um den Stab auf den Dorn (20) zu drücken;
f) Bearbeiten der Außenfläche (12) des Stabs mit einer CNC-Drehmaschine, um eine gewünschte Außenkontur des Gehäuses (10) zu erzeugen.

2. Verfahren gemäß Anspruch 1, worin das für medizinische Anwendung geeignete Polymer ausgewählt ist aus der aus Polycarbonat, Polyethylenterephthalat, Polymethylmethacrylat und Polypropylen bestehenden Gruppe.

3. Verfahren gemäß Anspruch 2, worin das für medizinische Anwendung geeignete Polymer Polycarbonat ist.

4. Verfahren gemäß Anspruch 1, worin das für medizinische Anwendung geeignete Polymer ein nicht-thermoplastisches Polymer ist.

5. Verfahren gemäß Anspruch 4, worin das für medizinische Anwendung geeignete Polymer ausgewählt ist aus der aus Polytetrafluorethylen, Silikonkautschuk und Polyurethan bestehenden Gruppe.

6. Verfahren gemäß einem der Ansprüche 1 bis 5, worin der Dorn (20) einen scheibenförmigen Sockel (21), einen Kegelstumpf (22), der mit der Basis (21) verbunden ist, und mindestens eine Gewindebohrung (23) in einer der Basis (21) gegenüberliegenden Fläche des Kegelstumpfs (22) umfasst.

7. Verfahren gemäß einem der Ansprüche 1 bis 6, worin die Befestigungsabdeckung (40) scheibenförmig ist und mehrere Bohrungen aufweist, die den gleichen Abstand zur Mitte der Scheibe haben.

8. Verfahren gemäß einem der Ansprüche 1 bis 7, das Verbindungselement (30) mindestens eine Schraube aufweist.

9. Verfahren gemäß einem der Ansprüche 1 bis 8, worin die Bohrung (11) die Form eines Kegelstumpfs mit einem Öffnungswinkel im Bereich von 2° bis 4° hat.

10. Verfahren gemäß einem der Ansprüche 1 bis 9, worin die Wandstärke des Gehäuses (10) nach Schritt f) im Bereich von 0.5 bis 5 mm beträgt.

11. Verfahren gemäß einem der Ansprüche 1 bis 10, worin die Wandstärke des Gehäuses (10) nach Schritt f) über die Länge des Gehäuses variiert.

12. Verfahren gemäß einem der Ansprüche 1 bis 11, worin die arithmetische durchschnittliche Rauheit Rₐ der Wandoberfläche des Gehäuses (10), gemessen nach DIN EN ISO 4287, nach Schritt f) im Bereich von 0,2 µm bis 5,0 µm beträgt.

13. Verfahren gemäß einem der Ansprüche 1 bis 12, worin das Medizinprodukt ein Kapillardialysator, ein Hämofilter, ein Plasmafilter oder ein Ultrafilter ist.

14. Verfahren gemäß einem der Ansprüche 1 bis 12, worin das Medizinprodukt eine Vorrichtung zur extrakorporalen Membranoxygenierung oder eine Vorrichtung zur extrakorporalen Kohlendioxidentfernung ist.

## Revendications

1. Procédé de fabrication d'un boîtier en plastique à symétrie de rotation (10) pour un dispositif médical, comprenant
a) l'obtention d'une tige cylindrique massive d'un polymère de qualité médicale ;
b) la production d'un alésage à symétrie de rotation (11) le long de l'axe longitudinal de la tige cylindrique, l'alésage (11) s'étendant d'une première extrémité de la tige cylindrique à une deuxième extrémité, opposée de la tige cylindrique, le diamètre de l'alésage (11) diminuant depuis la première extrémité en direction de la deuxième extrémité ;
c) l'introduction d'un mandrin (20) ayant une forme correspondant au contour interne de l'alésage (11) dans l'alésage (11) depuis la première extrémité de la tige ;
d) le montage d'un couvercle de fixation (40) sur la deuxième extrémité de la tige ;
e) l'assemblage du mandrin (20) et du couvercle de fixation (40) par une pièce de fixation (30) pour appuyer la tige sur le mandrin (20) ;
f) l'usinage de la surface externe (12) de la tige au moyen d'un tour CNC pour produire un contour externe souhaité du boîtier (10).

2. Procédé de la revendication 1, dans lequel le polymère de qualité médicale est choisi dans le groupe constitué par le polycarbonate, le téréphtalate de polyéthylène, le polyméthacrylate de méthyle, et le polypropylène.

3. Procédé de la revendication 2, dans lequel le polymère de qualité médicale est le polycarbonate.

4. Procédé de la revendication 1, dans lequel le polymère de qualité médicale est un polymère non thermoplastique.

5. Procédé de la revendication 4, dans lequel le polymère de qualité médicale est choisi dans le groupe constitué par le polytétrafluoroéthylène, le caoutchouc de silicone, et le polyuréthane.

6. Procédé de l'une quelconque des revendications 1 à 5, dans lequel le mandrin (20) comprend une base en forme de disque (21), un cône tronqué (22) relié à la base (21), et au moins un alésage fileté (23) dans une face du cône tronqué (22) à l'opposé de la base (21).

7. Procédé de l'une quelconque des revendications 1 à 6, dans lequel le couvercle de fixation (40) est en forme de disque et a plusieurs alésages équidistants par rapport au centre du disque.

8. Procédé de l'une quelconque des revendications 1 à 7, dans lequel la pièce de fixation (30) comprend au moins une vis.

9. Procédé de l'une quelconque des revendications 1 à 8, dans lequel l'alésage (11) a la forme d'un cône tronqué avec un angle d'ouverture dans la gamme de 2° à 4°.

10. Procédé de l'une quelconque des revendications 1 à 9, dans lequel l'épaisseur de paroi du boîtier (10) après l'étape f) se situe dans la gamme de 0,5 à 5 mm.

11. Procédé de l'une quelconque des revendications 1 à 10, dans lequel l'épaisseur de paroi du boîtier (10) après l'étape f) varie sur la longueur du boîtier.

12. Procédé de l'une quelconque des revendications 1 à 11, dans lequel la rugosité moyenne arithmétique Rₐ de la surface de paroi du boîtier (10), mesurée conformément à la norme DIN EN ISO 4287, après l'étape f) se situe dans la gamme de 0,2 µm à 5,0 µm.

13. Procédé de l'une quelconque des revendications 1 à 12, dans lequel le dispositif médical est un dialyseur capillaire, un hémofiltre, un filtre à plasma, ou un ultrafiltre.

14. Procédé de l'une quelconque des revendications 1 à 12, dans lequel le dispositif médical est un dispositif pour l'oxygénation membranaire extracorporelle ou un dispositif pour l'épuration extracorporelle du dioxyde de carbone.
